# EUROPEAN PATENT APPLICATION

(11) **EP 4 782 017 A1**
(43) Date of publication of application: **29.07.2026**
(21) Application number: 24868642.0
(22) Date of filing: 13.09.2024
(51) Int. Cl.: A61L 27/36

(54) **METHOD FOR PREPARING DECELLULARIZED MATRIX WITH ENHANCED BIOCOMPATIBILITY ON BASIS OF SUPERCRITICAL CARBON DIOXIDE PROCESS**

(30) Priority: 19.09.2023 KR 20230124815
(71) Applicant: Medifab Co., Ltd., Seoul 08594 (KR)
(72) Inventor: KIM, Su Hee, Seoul 08594 (KR); CHA, Mi Sun, Seoul 08594 (KR); KANG, Jae Jun, Seoul 08594 (KR)
(74) Representative: Morabito, Sara
(86) International application number: PCT/KR2024/014053
(87) International publication number: WO 2025/063665

(57) **Abstract**

According to a method according to an aspect, in a process of enhancing biocompatibility of a decellularized extracellular matrix (dECM) through supercritical carbon dioxide treatment, immunogenicity of the dECM may be reduced with excellent efficiency, while maintaining the content and physiological activity of bioactive substances.

Accordingly, a decellularized extracellular matrix (dECM) prepared according to a method according to an aspect, a composition comprising the same, and a biomaterial have low cytotoxicity and excellent tissue regenerative capability, and thus may be usefully used as cosmetic or medical materials.

## Description

### Technical Field

The present application relates to a method of preparing a decellularized extracellular matrix (dECM) with enhanced biocompatibility and physiological activity.

### Background Art

An extracellular matrix (ECM) is a non-cellular structure of a tissue or an organ, consisting of various polymeric substances secreted from cells. The extracellular matrix may be used as a natural scaffold material that is structurally sound and suitable for biological purposes. Its specific composition and function may vary depending on the specific form of tissue from which it originated.

A decellularization process is a process of separating extracellular matrix components from biological tissue through chemical treatment. Through the decellularization process, components with immunogenic properties, such as cellular components, are removed from biological tissue, whereas bioactive substances involved in implementing the environment and functions of biological tissue, such as extracellular matrix and some growth factor proteins, may be preserved. Accordingly, decellularized extracellular matrix (dECM) including various bioactive substances preserved as described above, for example, collagen, glycosaminoglycan, various cytokines, etc. (hereinafter, decellularized extracellular matrix (dECM)) promotes tissue regeneration and provides a more natural biomimetic microenvironment for cells to grow and differentiate. Such decellularized extracellular matrix (dECM) may be used alone or in combination with other materials for tissue regeneration or the like.

Meanwhile, in a decellularization process or in a process of degrading decellularized extracellular matrix (dECM) into a low molecular weight form or treating the same to remove immunogenicity, depending on reaction conditions including solvent, degrading enzyme, temperature conditions, pH conditions, etc., bioactive substances in the dECM may be removed or denatured, resulting in a reduction in physiological activity. In addition, existing methods using surfactants or the like may exhibit toxicity upon biological application since the dECM may include residual surfactants. In addition, depending on reaction conditions, the osmotic pressure of a composition including the dECM may increase, and as the osmotic pressure increases, the level of mixing with other materials may decrease, and the efficiency of compounding with other cosmetic or medical materials or components may decrease.

In addition, the extracellular matrix or the dECM contains collagen, and at both ends of a collagen molecule, there is a portion consisting of 12 to 27 amino acids, called a telopeptide. Such telopeptides are the main cause of immune responses, and therefore, to apply the dECM in vivo, a process of removing telopeptides is required. Meanwhile, existing processes have removed such telopeptide sequences by using an acidic solution, an enzyme, or a combination thereof. However, such existing methods involve chemical acid or base treatment that increases osmotic pressure, making compounding with other materials difficult, and may also have harmful effects on the human body due to additional treatment with acidic or alkaline solutions. In addition, such multi-step treatment processes are complicated, and there is a problem in that the efficacy upon biological application is reduced due to removal of active ingredients or bioactive substances in the extracellular matrix during the treatment process.

Accordingly, to solve such problems, the inventors of the present disclosure have developed a technique for preparing a decellularized extracellular matrix (dECM) material that, through reduction or removal of the content of telopeptides, exhibits a reduced immune response or does not induce an immune response upon application in vivo, while maintaining the content and physiological activity of bioactive substances in the dECM.

### Disclosure of Invention

### Technical Problem

An aspect is to provide a method of preparing a decellularized extracellular matrix (dECM) with enhanced biocompatibility, the method comprising treating a solution comprising a decellularized extracellular matrix (dECM) with supercritical carbon dioxide, wherein the treating with the supercritical carbon dioxide is performed at 60 to 150 °C.

Another aspect is to provide the dECM prepared by the method, a composition comprising the same, and a biomaterial comprising the same.

Another aspect is to provide a method of enhancing biocompatibility of a decellularized extracellular matrix (dECM), the method comprising treating a solution comprising a decellularized extracellular matrix (dECM) with supercritical carbon dioxide, wherein the treating with the supercritical carbon dioxide is performed at 60 to 150 °C.

Other objectives and advantages of the present application will become apparent from the following detailed description, together with the appended claims and drawings. Any matter not described in the present application is sufficiently recognizable and inferable to a person skilled in the art of the present application or a similar technical field, and therefore, its description is omitted.

### Solution to Problem

Throughout the present application, when a part "comprises" a certain component, this means that it may further comprise other components rather than excluding other components, unless specifically stated to the contrary. Additionally, unless a specific order is explicitly stated in the context, the respective processes may be performed in an order different from that stated. That is, the respective processes may be performed in the same order as stated, may be performed substantially simultaneously, or may be performed in the reverse order.

An aspect is to provide a method of preparing a decellularized extracellular matrix (dECM) with enhanced biocompatibility, the method comprising treating a solution comprising a decellularized extracellular matrix (dECM) with supercritical carbon dioxide, wherein the treating with the supercritical carbon dioxide is performed at 60 to 150 °C.

As used herein, "biocompatibility" refers to a substance introduced into an original cell, tissue, organ, or organism performing a desired function without inducing a significant inflammatory response, immunogenicity, or cytotoxicity to the cell, tissue, organ, or organism. For example, a biocompatible material refers to a material that performs a desired function when introduced into an organism without inducing a significant inflammatory response, immunogenicity, or cytotoxicity to the original cell, tissue, organ, or organism.

In an embodiment, the enhancement of biocompatibility may refer to a reduced inflammatory response, immunogenicity, or cytotoxicity exhibited upon biological application when a decellularized extracellular matrix (dECM) is treated with supercritical carbon dioxide according to an embodiment, compared to a case in which the treatment is not performed. For example, the enhancement of biocompatibility may comprise a decrease in the telopeptide content in the dECM.

As used herein, the term "decellularized extracellular matrix (dECM)" may be used interchangeably with "decellularized tissue," or "decellularized material." The dECM refers to a tissue or organ of a human or an animal such as a porcine or cattle that has undergone decellularization to remove cellular components other than the extracellular matrix (ECM), for example, nucleus, cell membrane, and nucleic acids, etc. Meanwhile, a "decellularized organ" refers to an organ, such as a heart or kidney, that has been decellularized while maintaining the overall structure of the organ, and is clearly distinguished from the "decellularized extracellular matrix (dECM)" of the present application in that it is intended to reimplant cells or to use the entire physical structure of the organ as is, and thus a soluble formulation of the extracellular matrix is not possible.

The extracellular matrix refers to a complex collection of biopolymers filling intra-tissue or extracellular spaces. The extracellular matrix is consisted of various types of molecules synthesized by cells, secreted, and accumulated extracellularly, such as fibrous proteins, complex proteins such as proteoglycans, and cell-adhesive proteins such as fibronectin and laminin, etc.

The method of preparing a decellularized extracellular matrix (dECM) with enhanced biocompatibility may not comprise treating an acid or alkaline solution. The method of preparing a decellularized extracellular matrix (dECM) with enhanced biocompatibility according to an embodiment comprises, unlike existing methods of treating an acid or alkaline solution, treating supercritical carbon dioxide performed at a specific temperature, and as an effect derived therefrom, does not comprise a subsequent treatment of an acid or alkaline solution and a subsequent treatment of a degrading enzyme, thereby achieving high efficiency in the manufacturing process and not requiring a separate purification process. Accordingly, the dECM prepared by the method according to an embodiment maintains the content of bioactive substances and their physiological activity, has low telopeptide content and low immunogenicity, and thus exhibits excellent biocompatibility.

In an embodiment, the treating with the supercritical carbon dioxide may be performed at 60 to 150 °C. The treating with the supercritical carbon dioxide may be performed in a reactor, wherein the temperature in the reactor is from 60 to 150 °C. For example, the treating with the supercritical carbon dioxide may be performed under conditions in which the temperature in the reactor is 60 to 150 °C, 60 to 145 °C, 60 to 140°C, 60 to 135 °C, 60 to 130°C, 60 to 125 °C, 60 to 120°C, 60 to 115 °C, 60 to 110 °C, 60 to 105 °C, 60 to 100 °C, 60 to 95 °C, 60 to 90 °C, 60 to 85 °C, 60 to 80 °C, 60 to 75 °C, 60 to 70 °C, 65 to 150 °C, 65 to 145 °C, 65 to 140°C, 65 to 135 °C, 65 to 130°C, 65 to 125 °C, 65 to 120°C, 65 to 115 °C, 65 to 110 °C, 65 to 105 °C, 65 to 100 °C, 65 to 95 °C, 65 to 90 °C, 65 to 85 °C, 65 to 80 °C, 65 to 75 °C, 65 to 70 °C, 70 to 150 °C, 70 to 145 °C, 70 to 140 °C, 70 to 135 °C, 70 to 130°C, 70 to 125 °C, 70 to 120°C, 70 to 115 °C, 70 to 110°C, 70 to 105 °C, 70 to 100 °C, 70 to 95 °C, 70 to 90 °C, 70 to 85 °C, 70 to 80 °C, 70 to 150°C, 70 to 145 °C, 70 to 140°C, 70 to 135 °C, 70 to 130°C, 70 to 125 °C, 70 to 120°C, 70 to 115 °C, 70 to 110 °C, 70 to 105 °C, 70 to 100 °C, 70 to 95 °C, or 70 to 90 °C, but is not limited thereto. If the temperature falls outside the above range, the dECM may not be sufficiently depolymerized or hydrolyzed, and there is a concern that the telopeptide content may not be sufficiently reduced.

In an embodiment, the treating with the supercritical carbon dioxide may be performed in a reactor, and carbon dioxide may be injected so that a pressure in the reactor becomes 70 to 400 bar. For example, carbon dioxide gas may be injected into the reactor so that a pressure in the reactor becomes 80 to 400 bar, 90 to 400 bar, 100 to 400 bar, 110 to 400 bar, 120 to 400 bar, 130 to 400 bar, 140 to 400 bar, 150 to 400 bar, 160 to 400 bar, 170 to 400 bar, 180 to 400 bar, 190 to 400 bar, 150 to 390 bar, 150 to 380 bar, 150 to 370 bar, 150 to 360 bar, 150 to 350 bar, 160 to 350 bar, 170 to 350 bar, 180 to 350 bar, 190 to 350 bar, 200 to 350 bar, 210 to 350 bar, 220 to 350 bar, 230 to 350 bar, 240 to 350 bar, 250 to 350 bar, 260 to 350 bar, 270 to 350 bar, 280 to 350 bar, 290 to 350 bar, or 300 to 350 bar, but is not limited thereto. If the pressure falls outside the above range and a pressure in the reactor is low, there is a concern that the dECM may not be sufficiently depolymerized or hydrolyzed, or that the telopeptide content may not be sufficiently reduced.

In an embodiment, the treating with the supercritical carbon dioxide may be performed for 1 to 48 hours, and a pressure in the reactor may be maintained for 1 to 48 hours. For example, the treating with the supercritical carbon dioxide may be performed for 1 to 36 hours, 1 to 30 hours, 1 to 24 hours, 1 to 18 hours, 6 to 36 hours, 6 to 30 hours, 6 to 24 hours, 6 to 18 hours, 6 to 17 hours, 6 to 16 hours, or 6 to 15 hours, but is not limited thereto. If the treating with the supercritical carbon dioxide is shorter than the above time, there is a concern that the dECM may not be sufficiently depolymerized or hydrolyzed, resulting in insufficient reduction in the telopeptide content.

In an embodiment, the solution comprising the dECM may be a solution comprising a dECM powder. The powder of the dECM may be a dried powder of an extracellular matrix that has undergone a decellularization process, and may be a powder obtained by drying the extracellular matrix that has undergone the decellularization process and pulverizing the same. For example, it may be obtained by drying a decellularized extracellular matrix (dECM) that has undergone the decellularization process and then pulverizing the same. The drying method of preparing the extracellular matrix in a dry state may employ methods generally used in the art, and is not particularly limited. Non-limiting examples of the drying method comprise an air-drying method, a natural drying method, a spray drying method, a lyophilization method, and a reduced pressure drying method, etc. These methods may be used alone or in combination of at least two methods. In an embodiment, the dried powder of the dECM may be a lyophilized powder of the dECM. The addition of the powder of the dECM and distilled water to the reactor and the injection of supercritical carbon dioxide may be performed simultaneously or sequentially.

As used herein, the term "solution" may refer to a state in which two substances are mixed, for example, a state in which a solute and a solvent are mixed. In an embodiment, the solution comprising the dECM powder may refer to a mixture, suspension, or aqueous solution in which the dECM powder is comprised in distilled water. As used herein, the term "solution" may be used interchangeably with mixture, suspension, hydrate, composition, melt, aqueous solution, etc.

In an embodiment, the solution comprising the dECM may comprise the dECM powder, may comprise the powder of the dECM, and distilled water. The solution comprising the dECM powder may comprise 0.1 to 5 wt% of the dECM powder. In an embodiment, the powder of the dECM may be comprised in distilled water in an amount of 0.01 to 5 wt%, and for example, 0.05 to 5 wt%, 0.1 to 5 wt%, 0.2 to 5 wt%, 0.3 to 5 wt%, 0.4 to 5 wt%, 0.5 to 5 wt%, 0.6 to 5 wt%, 0.7 to 5 wt%, 0.8 to 5 wt%, 0.9 to 5 wt%, 1.0 to 5 wt%, 0.05 to 4 wt%, 0.1 to 4 wt%, 0.2 to 4 wt%, 0.3 to 4 wt%, 0.4 to 4 wt%, 0.5 to 4 wt%, 0.6 to 4 wt%, 0.7 to 4 wt%, 0.8 to 4 wt%, 0.9 to 4 wt%, 1.0 to 4 wt%, 0.05 to 3 wt%, 0.1 to 3 wt%, 0.2 to 3 wt%, 0.3 to 3 wt%, 0.4 to 3 wt%, 0.5 to 3 wt%, 0.6 to 3 wt%, 0.7 to 3 wt%, 0.8 to 3 wt%, 0.9 to 3 wt%, or 1.0 to 3 wt%, but is not limited thereto.

In an embodiment, the dECM may have a reduced content of telopeptides in the dECM or may have telopeptides removed.

As used herein, a reduction in the content of telopeptides or removal of telopeptides refers to the content of telopeptides comprised in a decellularized extracellular matrix (dECM) being reduced as compared with that before performing the treatment with supercritical carbon dioxide according to an aspect. For example, by treating the solution comprising the dECM with supercritical carbon dioxide under temperature conditions of 60 to 150 °C, the content of telopeptides in the dECM may be reduced by 80 % or more. For example, the content of telopeptides comprised in the dECM may be reduced by 60 % or more, 65 % or more, 70 % or more, 75 % or more, or 80 % or more, by performing the treating with the supercritical carbon dioxide according to an embodiment. The dECM may have enhanced biocompatibility by reducing the content of telopeptides in the dECM or by removing telopeptides.

According to a method according to an embodiment, it was confirmed that the telopeptide content may be reduced with an efficiency similar to or higher than that of existing methods, compared to a method of treating an existing acidic solution, an alkaline solution, or an acidic solution and an enzyme.

In an embodiment, the dECM may comprise a high content of a bioactive substance or active ingredient. For example, the dECM may refer to a matrix in which, even after treatment with supercritical carbon dioxide under temperature conditions of 60 to 150 °C, the content of bioactive substances or active ingredients in the dECM is maintained at 80 % or more as compared with that before the treatment, for example, 80 %, 81 %, 82 %, 83 %, 84 %, 85 %, 86 %, 87 %, 88 %, 89 %, 90 %, 91 %, 92 %, 93 %, 94 %, or 95 % or more. According to a method according to an embodiment, it was confirmed that, compared to the case of treating existing acidic solutions, alkaline solutions, or acidic solutions and enzymes, the residual amount of bioactive substances or active ingredients in the dECM was significantly higher. For example, it was confirmed that the residual amounts of collagen and elastin in the dECM were each 95 % or more. This confirmed that, according to a method according to an embodiment, it is possible to prepare a decellularized extracellular matrix (dECM) having excellent physiological activity containing a large amount of bioactive substances.

Accordingly, according to the method according to an embodiment, it may be seen that not only may the immunogenicity of the dECM be efficiently reduced to enhance biocompatibility, but also at the same time, the active ingredients or bioactive substances in the dECM may be maintained at a high content to prepare a decellularized extracellular matrix (dECM) having excellent physiological activity.

In an embodiment, the matrix may be an extracellular matrix derived from any one selected from the group consisting of skin tissue, cardiac tissue, adipose tissue, corneal tissue, bone tissue, brain tissue, vascular tissue, or cartilage tissue of humans or animals such as porcine or cattle, etc. but is not limited thereto.

Meanwhile, the method may be applied without limitation to techniques known in the art related to methods for decellularizing extracellular matrix.

For example, as a method of decellularizing the extracellular matrix, the dECM may have been decellularized by a decellularization method comprising an injection of supercritical carbon dioxide into a reactor comprising biological tissue and ethanol so that a pressure in the reactor becomes 70 to 400 bar, and the injection of the supercritical carbon dioxide into the reactor comprising the biological tissue and ethanol may be performed at 31 to 60 °C.

The decellularization method may comprise, for example, an injection of carbon dioxide gas into the reactor to maintain a pressure in the reactor at 80 to 400 bar, 90 to 400 bar, 100 to 400 bar, 110 to 400 bar, 120 to 400 bar, 130 to 400 bar, 140 to 400 bar, 150 to 400 bar, 160 to 400 bar, 170 to 400 bar, 180 to 400 bar, 190 to 400 bar, 150 to 390 bar, 150 to 380 bar, 150 to 370 bar, 150 to 360 bar, 150 to 350 bar, 160 to 350 bar, 170 to 350 bar, 180 to 350 bar, 190 to 350 bar, 200 to 350 bar, 210 to 350 bar, 220 to 350 bar, 230 to 350 bar, 240 to 350 bar, 250 to 350 bar, 260 to 350 bar, 270 to 350 bar, 280 to 350 bar, 290 to 350 bar, or 300 to 350 bar, but is not limited thereto.

The decellularization method may comprise, for example, treating the supercritical carbon dioxide under conditions in which the temperature in the reactor is 31 to 55 °C, 31 to 50 °C, 31 to 45 °C, 31 to 40 °C, 31 to 39 °C, 31 to 38 °C, 31 to 37 °C, 32 to 40 °C, 33 to 40 °C, 34 to 40 °C, 35 to 40 °C, 32 to 39 °C, 33 to 39 °C, 34 to 39 °C, 35 to 39 °C, 32 to 38 °C, 33 to 38 °C, 34 to 38 °C, 35 to 38 °C, 32 to 37 °C, 33 to 37 °C, 34 to 37 °C, or 35 to 37 °C, but is not limited thereto.

The decellularization method may be a method of treating the supercritical carbon dioxide for 2 to 24 hours, and for example, may be a method of maintaining a pressure in the reactor for 2 to 24 hours. For example, a pressure in the reactor may be maintained for 3 to 23 hours, 4 to 22 hours, 5 to 21 hours, 6 to 20 hours, 6 to 19 hours, 6 to 18 hours, 6 to 17 hours, 6 to 16 hours, 6 to 15 hours, 6 to 14 hours, 6 to 13 hours, or 6 to 12 hours, but is not limited thereto.

The decellularization method may not comprise treatment with an additional acid, base, or enzyme. The decellularization method may additionally comprise a pretreatment prior to bringing the biological tissue into contact with ethanol in the reactor. Through the pretreatment process, contaminants, fat, etc. of biological tissue may be removed. In addition, the decellularization method may further comprise treatment of the mixture, which has undergone an injection of supercritical carbon dioxide into the reactor comprising the biological tissue and ethanol, with a DNA decomposing enzyme, which serves to remove the immunogenicity of the mixture by decomposing the DNA remaining in the mixture. The decellularization method may further comprise a washing process of treating the mixture, which has undergone the process of treating with the DNA decomposing enzyme, with distilled water to remove residual contaminants.

Another aspect is to provide a decellularized extracellular matrix (dECM) prepared by the method. In the dECM, the same terms or elements mentioned above as those already mentioned are as mentioned above.

Another aspect is to provide a composition comprising the dECM. In the composition, the same terms or elements as those already mentioned are as described above.

In an embodiment, the composition may be a hydrogel composition comprising the dECM. For example, the hydrogel composition may be a hydrogel composition comprising the dECM according to an embodiment and hyaluronic acid, or a hydrogel composition comprising the dECM according to an embodiment and chitosan. The hydrogel composition may further comprise any other material suitable for preparing a hydrogel composition.

As used herein, the term "hydrogel" may refer to a three-dimensional network structure formed by cross-linking a hydrophilic polymer through covalent or noncovalent bonds. Due to the hydrophilicity of the constituent materials, it absorbs and swells in a large amount of water in an aqueous solution and aqueous environment but has the property of not dissolving due to the cross-linked structure. Therefore, various forms and properties of hydrogels may be prepared depending on constituent components and a manufacturing method, and since in general, a hydrogel contains a large amount of moisture and may have intermediate properties between a liquid and a solid.

As used herein, the term "chitosan" may refer to a linear polysaccharide consisting of D-glucosamine and N-acetylglucosamine. The chitosan may comprise, in addition to pure chitosan, chitosan derivatives. For example, the chitosan derivatives may comprise at least any one of methylated chitosan, carboxymethylated chitosan, phthalated chitosan, esterified chitosan, amidated chitosan, or formylated chitosan.

In an embodiment, the composition may be a composition for tissue repair, which may be a material used for tissue repair. For example, it may refer to a filler material similar to soft tissue injected into skin having wrinkles or into a region requiring volume, an adhesion-preventing material between a surgical site and normal tissue, a tissue adhesive material, or a wound covering material for artificial skin, etc. The hydrogel may be applied to, for example, glabella, forehead, under-eye aegyo-sal, crow's feet, nasolabial folds, cheek, perioral wrinkles, and chin, etc. The hydrogel must be biocompatible as it is a material that is directly applied to the human body. For example, when a hydrogel is filled into a region requiring volume, excellent retention/persistence of the gel shape is required to form volume for a long period after injection. When the hydrogel is used for adhesion prevention at a surgical site, it must have tissue compatibility at a wound site and have low or no cytotoxicity. When used as a wound dressing material, excellent holding power/persistence is required to maintain the adhesion and application state. In addition, depending on the hydrogel treatment conditions, the skin surface may become uneven or the treatment result may be undesirable, and therefore, the hydrogel formed in the body must be easily decomposable or modifiable.

The composition may have enhanced biocompatibility and physiological activity by comprising a decellularized extracellular matrix (dECM) according to an embodiment, and thus may be usefully used as a composition for tissue repair.

Another aspect is to provide a biomaterial comprising the dECM. In the biomaterial, the same terms or elements mentioned above, as those already mentioned are as above.

In an embodiment, the biomaterial may be a biomaterial comprising the hydrogel composition.

In an embodiment, the biomaterial may be a biomaterial for tissue repair.

The biomaterial may have enhanced biocompatibility and physiological activity by comprising a decellularized extracellular matrix (dECM) according to an embodiment, and thus may be usefully used as a biomaterial for tissue repair.

Another aspect is to provide a method of enhancing biocompatibility of a decellularized extracellular matrix (dECM), the method comprising treating a solution comprising a decellularized extracellular matrix (dECM) with supercritical carbon dioxide, wherein the treating with the supercritical carbon dioxide is performed at 60 to 150 °C. In the method of enhancing the biocompatibility of the dECM, the same terms or elements as those already mentioned are as described above.

### Advantageous Effects of Invention

According to a method according to an aspect, not only may the biocompatibility of the dECM be improved, but also the content and physiological activity of the bioactive substance may be maintained.

Accordingly, the dECM prepared according to the method according to an aspect has low cytotoxicity and may be applied in vivo due to high biocompatibility and physiological activity, and thus may be usefully employed as a biomaterial for tissue repair.

### Brief Description of Drawings

FIG. 1A shows the result of treating a decellularized extracellular matrix (dECM) according to an embodiment through a supercritical carbon dioxide process.
FIG. 1B shows the result of treating a decellularized extracellular matrix (dECM) according to an embodiment by using an HCl solution.
FIG. 1C shows the result of treating a decellularized extracellular matrix (dECM) according to an embodiment by using a NaOH solution.
FIG. 2A shows the result of treating a decellularized extracellular matrix (dECM) according to an embodiment through a supercritical carbon dioxide process.
FIG. 2B shows the result of treating a decellularized extracellular matrix (dECM) according to an embodiment by using an HCl solution.
FIG. 2C shows the result of treating a decellularized extracellular matrix (dECM) according to an embodiment using a NaOH solution.
FIG. 3 shows the results of absorbance measurements used to determine and compare the contents of telopeptides in decellularized extracellular matrix (dECM) for different treatment methods.
FIG. 4 shows the results of a hydroxyproline assay and an elastin assay used to determine and compare the contents of residual active ingredients in decellularized extracellular matrix (dECM) for different treatment methods.
FIG. 5 shows the results of confirming the degree of occurrence of an immune response after injecting, into mouse skin, a composition comprising decellularized material according to an embodiment.

### Mode for the Invention

The present disclosure will be explained in more detail in the following examples. However, these examples are for illustrative purposes only and the scope of the present disclosure is not limited to these examples.

### Preparation Example 1. Preparation of Decellularized Extracellular Matrix (dECM) through Supercritical Carbon Dioxide Process

Porcine skin tissue was prepared, the dead skin cells were removed with a knife, and a pretreatment process of removing fats was performed by stirring with 70 % ethanol at room temperature for 4 hours. Thereafter, 20 g of the skin tissue and 100 ml of 100 % ethanol were placed in a reactor of a supercritical fluid equipment (Ilshin Autoclave, Korea), and the temperature was set to 35 °C. Thereafter, carbon dioxide was injected to adjust the pressure to 300 bar, and the reaction was performed for 6 hours under the above temperature and pressure conditions. Afterwards, the mixture was washed with an ethanol solution for 2 hours, and washed with PBS and distilled water for 4 hours. The washed tissue was lyophilized and then pulverized using a freeze-miller.

### Example 1. Preparation of Decellularized Extracellular Matrix (dECM) Material with Enhanced Biocompatibility Based on Supercritical Carbon Dioxide Process

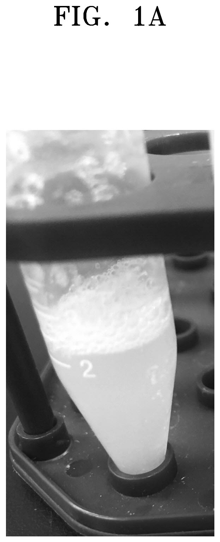

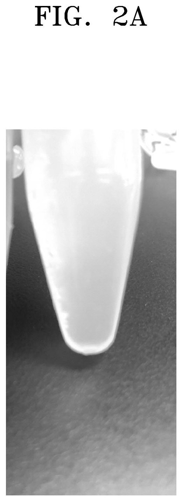

The dried powder of a decellularized extracellular matrix (dECM) prepared as in Preparation Example 1 was mixed in distilled water at 2.0 wt%, placed in a reactor of a supercritical fluid equipment (Ilshin Autoclave, Korea), and the temperature was set to 60 °C. Thereafter, carbon dioxide was injected to maintain a pressure in the reactor at 300 bar, and the reaction was performed for 12 hours under these conditions. The results of visual inspection of the dECM material prepared in this manner are shown in FIGS. 1A and 2A.

### Comparative Example 1. Preparation of Decellularized Extracellular Matrix (dECM) Material Using Acid

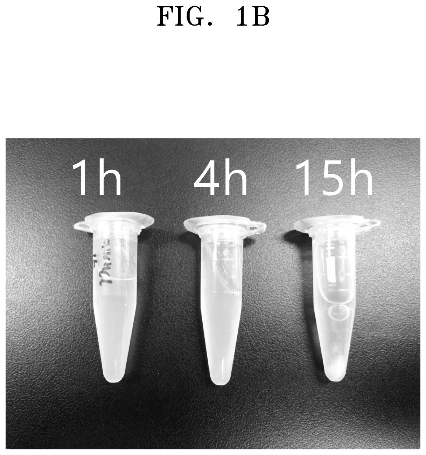

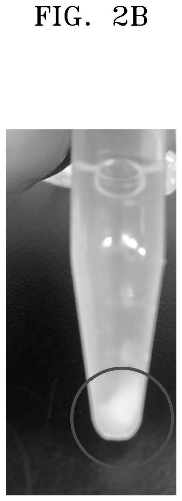

The dried powder of the dECM prepared as in Preparation Example 1 was mixed in a 0.1N HCl solution at 2.0 wt%. Thereafter, the temperature of the solution was adjusted to 60 °C and the reaction was performed for 1 hour, 4 hours, and 15 hours. The results of visual inspection of the dECM material prepared in this manner are shown in FIGS. 1B and 2B.

### Comparative Example 2. Preparation of Decellularized Extracellular Matrix (dECM) Material Using Base

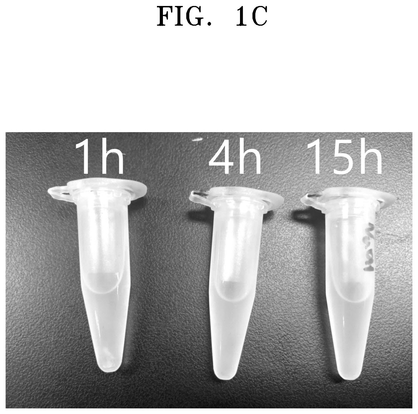

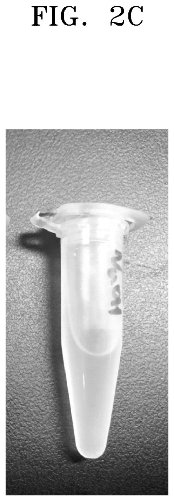

The dried powder of the dECM prepared as in Preparation Example 1 was mixed in a 0.2N NaOH solution at 2.0 wt%. Thereafter, the temperature of the solution was adjusted to 60 °C and the mixture was reacted for 1 hour, 4 hours, and 15 hours. The results of visual inspection of the dECM material prepared in this manner are shown in FIGS. 1C and 2C.

### Comparative Example 3. Preparation of Decellularized Extracellular Matrix (dECM) Material Using Acid and Enzyme

Pepsin was mixed in a 0.1N HCl solution at 10 wt% relative to the dECM, and then the dried powder of the dECM prepared as in Preparation Example 1 was mixed in at 2.0 wt%. Thereafter, the solution was reacted at 4 °C for 24 hours, and the solution was filtered through a 100 kDa filter.

### Experimental Example 1. Confirmation of Depolymerization and Formulation of Decellularized Extracellular Matrix (DECM) Material

To confirm the depolymerization and formulation of the dECM material according to the supercritical carbon dioxide process according to an example, the degree of depolymerization and formulation over time of the dECM materials of Example 1, Comparative Example 1, and Comparative Example 2 were confirmed.

As shown in FIG. 2B, it was confirmed that a precipitation phenomenon occurred when the dECM was treated using an acidic solution. In contrast, when using a supercritical carbon dioxide process or a base according to an example, it was confirmed that the dECM powder was uniformly dispersed and dissolved in the solution without any precipitation (FIGS. 2A and 2C). This confirmed that, by treating the dECM according to the method of an example, depolymerization of the dECM and stable formulation may be achieved without additional treatment with an acid, a base, or an enzyme.

### Experimental Example 2. Confirmation of Immµnogenicity Removal Efficiency of Supercritical Carbon Dioxide Process

To confirm the immunogenicity removal efficiency of the supercritical carbon dioxide process on the dECM according to an example, the degree of removal of telopeptides was measured using a Sandwich ELISA (enzyme-linked immunosorbent assay) as follows.

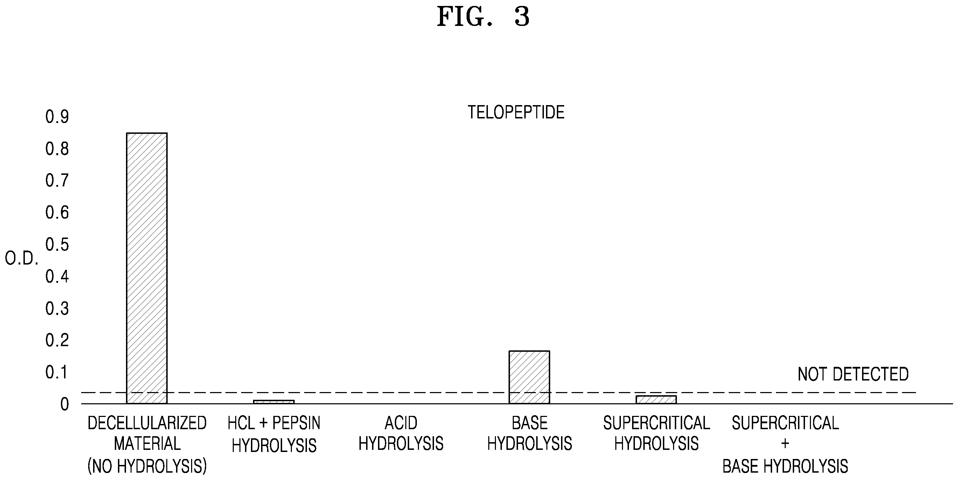

Specifically, a telopeptide standard solution sample, decellularized extracellular matrix (dECM) material samples according to Comparative Examples 1 to 3, a decellularized extracellular matrix (dECM) material sample according to Example 1, and the dECM material sample that was subjected to a supercritical carbon dioxide process (300 bar, 80 °C, 16 hours of reaction) in a similar manner to Example 1 and then additionally treated with a NaOH solution (0.2 N) (80 °C, 8 hours of reaction) ('supercritical + base hydrolysis' in Fig. 3) were each dispensed into a 96-well plate coated with a capture antibody. Thereafter, the mixture was reacted at 37 °C for 90 minutes and washed using a wash buffer. After treatment with a biotin-labeled detection antibody and reaction at 37 °C for 60 minutes, washing was performed using a wash buffer. After treatment with a Horseradish peroxidase-streptavidin (HRP-streptavidin) conjugate solution. The mixture were reacted at 37 °C for 30 minutes and then washed. The reaction was treated with a 3,3',5,5'-Tetramethylbenzidine (TMB) matrix solution and reacted at 37 °C for 15 minutes, after which a stop solution was added to stop the reaction. Thereafter, the absorbance value at a wavelength of 450 nm was measured and the telopeptide content in the samples was calculated using a standard curve.

As a result, as shown in Fig. 3, when the dECM was treated using a base, it was confirmed that the telopeptide sequence was detected at a high level, and in this case, it was confirmed that the telopeptide removal efficiency was reduced. In contrast, when the supercritical carbon dioxide process according to an embodiment was used, it was confirmed that the telopeptide sequence was not detected or was detected at a very low level. This confirmed that the supercritical carbon dioxide process according to an embodiment might efficiently remove telopeptides without additional treatment with an acid, a base, or an enzyme. This shows that the supercritical carbon dioxide process according to an embodiment may effectively remove immunogenicity of the dECM, thereby enhancing biocompatibility.

### Experimental Example 3. Confirmation of Residual Active Ingredient Content of Decellularized Material According to Treatment Method

To confirm the content of residual active ingredients after treating the dECM material using the supercritical carbon dioxide process according to an embodiment, a hydroxyproline assay and an elastin assay were performed as follows.

### (3-1). Performing Hydroxyproline Assay to Measure Residual Collagen

Samples of decellularized extracellular matrix (dECM) materials according to Comparative Examples 1 to 3 and Example 1 were mixed with 12N HCl at a ratio of 1:1 and hydrolyzed at 100 °C for 3 hours. Hydroxyproline standard solutions and the hydrolyzed samples were dispensed into a 96-well plate and the solvent was completely evaporated. Afterwards, Chloramine-T reagent was added to the wells into which the samples were dispensed and was reacted for 5 minutes at room temperature. After the reaction was completed, DMAB reagent was added and reacted at 60 °C for 90 minutes. After the reaction was completed, the absorbance was measured at a wavelength of 560 nm. The hydroxyproline content of the sample was calculated by constructing a hydroxyproline standard curve, and then quantified by converting it to collagen content.

### (3-2). Performino Elastin Assay to Measure Residual Elastin

Solutions of decellularized extracellular matrix (dECM) materials according to Comparative Examples 1 to 3 and Example 1 were mixed with oxalic acid and hydrolyzed at 100 °C. A supernatant comprising elastin was obtained using a centrifuge. An elastin precipitation reagent was added to the supernatant sample and elastin standard solution obtained in this manner and after incubation at 4 °C, an elastin precipitate was obtained using a centrifuge. Thereafter, staining reagent was added, treated at 4 °C, and centrifuged to obtain stained elastin pellets. After treating the samples comprising the pellets with a stain dissociation reagent, absorbance was measured at a wavelength of 513 nm. The elastin content was calculated using an elastin standard curve.

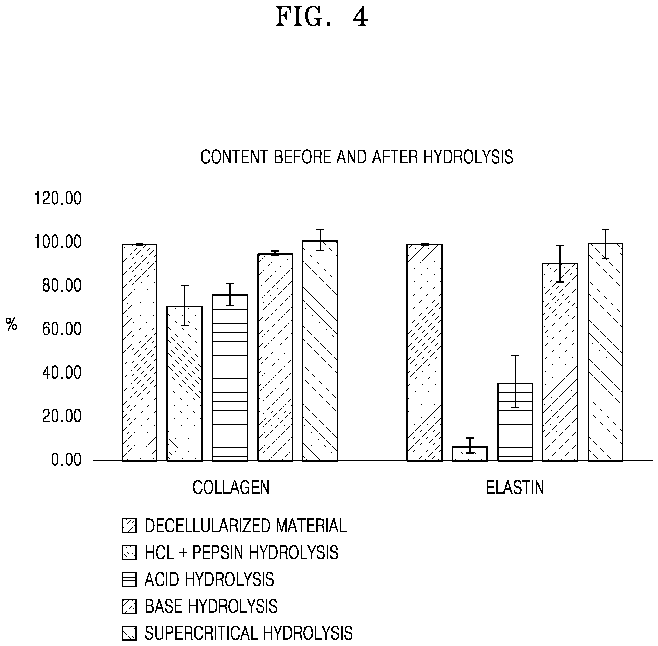

As a result, as shown in Fig. 4, in the case of Comparative Examples 1 and 2 using an acid or a base ('acid hydrolysis' and 'base hydrolysis' in FIG. 4, respectively), and Comparative Example 3 using an acid and an enzyme ('HCl+pepsin hydrolysis' in FIG. 4), it was confirmed that the amount of collagen and elastin remaining in the dECM material after hydrolysis was reduced. In comparison, in the case of the dECM material according to Example 1, it was confirmed that the amount of collagen and elastin was maintained at 95 % or more even after hydrolysis. This confirmed that when a decellularized extracellular matrix (dECM) material is treated through a supercritical carbon dioxide process according to an embodiment, the amount of residual active ingredients in the dECM is significantly greater than when treated with an acid, a base, or an enzyme, confirming that a bioactive substance in the extracellular matrix may be delivered into the body with excellent efficiency. This shows that, according to a method according to an embodiment, the content and physiological activity of a bioactive substance in a decellularized extracellular matrix (dECM) may be maintained.

### Experimental Example 4. Confirmation of Biocompatibility of Decellularized Material According to Example

To confirm the biocompatibility of the decellularized material prepared through the supercritical carbon dioxide process according to an embodiment, the degree of immune response occurrence upon in vivo application of the dECM was measured as follows.

### (4-1). Preparation of Samples

First, a 2 w/v% solution (dECM) of the dECM material according to Example 1, a solution (LTG-S Dermal filler) comprising 2 w/v% carboxymethyl chitosan mixed with 0.5 w/v% of the dECM material according to Example 1, and a 2 w/v% hyaluronic acid hydrogel sample (HA filler) were prepared.

Thereafter, 100 µl of each of the sample compositions (HA filler, LTG-S Dermal filler, dECM) was injected intradermally into mice, and tissues were collected from the mice 1 day and 7 days later, pathological staining was performed, and the following items were measured under a microscope.

### (4-2). Confirmation of Biocompatibility of Decellularized Extracellular Matrix (dECM)

To confirm the biocompatibility of the dECM treated by the method according to an embodiment, the number of inflammatory cells and the number of multinucleated cells that migrated into the sample injection site were analyzed. Additionally, the amount of collagen deposited into the sample injection site was measured in terms of area, the amount of new blood vessels and adipocytes induced into the sample injection site was measured, and the degree of necrosis of muscle cells around the sample injection site was measured.

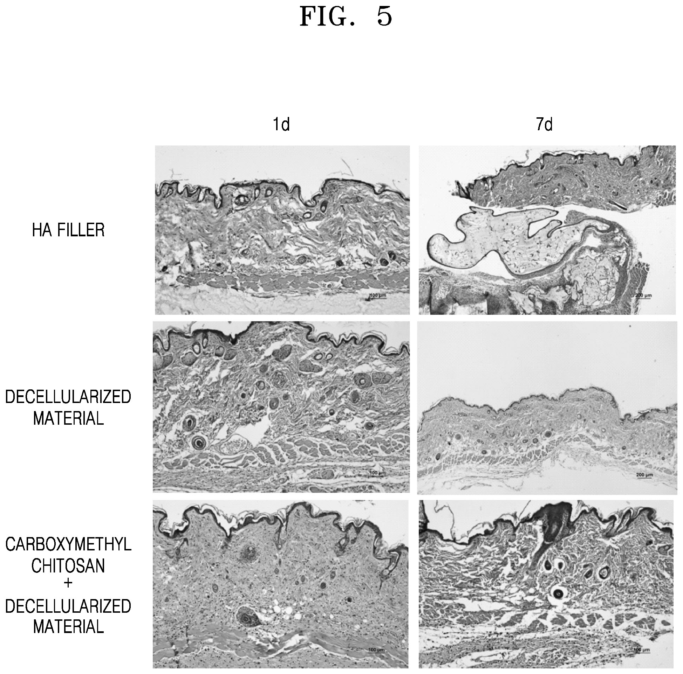

The results are shown in FIG. 5, Table 1, and Table 2.

**[Table 1]**

| Group | Measurement time point | Inflammatory cell infiltration | Number of multinucleated cells |
|---|---|---|---|
| HA filler | 1d | 0.0 ± 0.0 | 0.0 ± 0.0 |
| | 7d | 3.0 ± 1.4 | 0.0 ± 0.0 |
| LTG-S Dermal filler | 1d | 3.0 ± 0.0 | 0.0 ± 0.0 |
| | 7d | 2.0 ± 0.0 | 0.0 ± 0.0 |
| dECM | 1d | 0.0 ± 0.0 | 0.0 ± 0.0 |
| | 7d | 0.0 ± 0.0 | 0.0 ± 0.0 |

As shown in Table 1, when a decellularized extracellular matrix (dECM) or a hydrogel composite comprising the same prepared by a method according to an embodiment was treated, it was confirmed that inflammatory cell infiltration occurred to an insignificant extent, and no multinucleated cells were detected. This confirmed that the dECM or composite did not induce a significant immune response. Accordingly, it may be seen that the dECM according to an embodiment exhibits excellent biocompatibility.

**[Table 2]**

| Group | | Collagen deposition | Neovascularization | Adipocyte infiltration | Muscle cell necrosis |
|---|---|---|---|---|---|
| HA filler | 1d | 1.0 ± 0.0 | 0.0 ± 0.0 | 0.0 ± 0.0 | 0.0 ± 0.0 |
| | 7d | 3.0 ± 0.0 | 1.7 ± 0.6 | 1.3 ± 2.3 | 0.0 ± 0.0 |
| LTG-S Dermal filler | 1d | 3.0 ± 0.0 | 1.0 ± 0.0 | 0.0 ± 0.0 | 0.0 ± 0.0 |
| | 7d | 2.0 ± 0.0 | 0.5 ± 0.7 | 0.0 ± 0.0 | 0.0 ± 0.0 |
| dECM | 1d | 0.0 ± 0.0 | 0.0 ± 0.0 | 0.0 ± 0.0 | 0.0 ± 0.0 |
| | 7d | 0.0 ± 0.0 | 0.0 ± 0.0 | 0.0 ± 0.0 | 0.0 ± 0.0 |

As shown in Table 2, in the case of the dECM prepared by the method according to an embodiment and the hydrogel composite comprising the same, low levels of collagen deposition and neovascularization were observed. In addition, adipocyte infiltration and muscle cell necrosis were not observed. This confirmed that the dECM according to an embodiment did not exhibit a significant immune response, indicating that it exhibits excellent biocompatibility.

The foregoing description of the present disclosure is for illustrative purposes only, and one that has ordinary skill in the art to which the present disclosure belongs will understand that the present disclosure may be readily adapted to other specific forms without altering the technical ideas or essential features of the present disclosure. Therefore, the examples described above should be understood in all respects as illustrative and not restrictive.

## Claims

1. A method of preparing a decellularized extracellular matrix (dECM) with enhanced biocompatibility, the method comprising treating a solution comprising a decellularized extracellular matrix (dECM) with supercritical carbon dioxide, wherein the treating with the supercritical carbon dioxide is performed at 60 to 150 °C.

2. The method of claim 1, wherein the treating with the supercritical carbon dioxide is performed in a reactor and carbon dioxide is injected to maintain a pressure in the reactor at 70 to 400 bar.

3. The method of claim 1, wherein the treating with the supercritical carbon dioxide is performed for 1 to 48 hours.

4. The method of claim 1, wherein the solution comprises 0.1 to 5 wt% of decellularized extracellular matrix (dECM) powder.

5. The method of claim 1, wherein a content of telopeptides in the decellularized extracellular matrix (dECM) is reduced or removed.

6. The method of claim 1, wherein the decellularized extracellular matrix (dECM is derived from any one selected from the group consisting of skin tissue, cardiac tissue, adipose tissue, corneal tissue, bone tissue, brain tissue, vascular tissue, and cartilage tissue.

7. The method of claim 1, wherein the decellularized extracellular matrix (dECM) is prepared by a decellularization method comprising injecting supercritical carbon dioxide into a reactor comprising biological tissue and ethanol to maintain a pressure in the reactor at 70 to 400 bar, wherein the injection of the supercritical carbon dioxide into the reactor comprising the biological tissue and ethanol is performed at 31 to 60 °C.

8. A decellularized extracellular matrix (dECM) prepared by the method of claim 1.

9. The decellularized extracellular matrix (dECM) of claim 8, wherein a content of telopeptides in the decellularized extracellular matrix (dECM) is reduced or removed.

10. A composition comprising the decellularized extracellular matrix (dECM) of claim 8.

11. A biomaterial comprising the decellularized extracellular matrix (dECM) of claim 8.

12. A method of enhancing biocompatibility of a decellularized extracellular matrix (dECM), the method comprising treating a solution comprising a decellularized extracellular matrix (dECM) with supercritical carbon dioxide, wherein the treating with the supercritical carbon dioxide is performed at 60 to 150 °C.

13. The method of claim 12, wherein the treating with the supercritical carbon dioxide is performed in a reactor, and comprises injecting carbon dioxide is to maintain a pressure in the reactor at 70 to 400 bar.

14. The method of claim 12, wherein the treating with the supercritical carbon dioxide is performed for 1 to 48 hours.

15. The method of claim 12, wherein the decellularized extracellular matrix (dECM) is derived from any one selected from the group consisting of skin tissue, cardiac tissue, adipose tissue, corneal tissue, bone tissue, brain tissue, vascular tissue, and cartilage tissue.
